# EUROPEAN PATENT SPECIFICATION

(11) **EP 3 085 393 B1**
(45) Date of publication and mention of the grant of the patent: **25.11.2020**
(21) Application number: 16275057.4
(22) Date of filing: 20.04.2016
(51) Int. Cl.: A61L 9/14, A61L 9/12

(54) **FRAGRANCE DISPENSER**
DUFTSTOFFSPENDER
DISTRIBUTEUR D'ODEURS

(30) Priority: 21.04.2015 GB 201506748
(43) Date of publication of application: 26.10.2016
(73) Proprietor: Personnel Hygiene Services Limited, Caerphilly Gwent CF83 1XH (GB)
(72) Inventor: TODD, Jon, Caerphilly, Gwent CF83 1XH (GB); COTTON, Carl, Caerphilly, Gwent CF83 1XH (GB)
(74) Representative: Pawlyn, Anthony Neil

(56) References cited:
- EP-A1- 2 767 774
- WO-A1-2013/159142
- WO-A1-2015/050198

## Description

The present invention relates to a fragrance dispensing apparatus, and in particular to a device for including a piezo electric atomiser for dispensing a liquid fragrance aerosol spray.

Air freshening apparatuses are commonly provided in environments such as public washrooms to dispense an aerosol of liquid fragrance to provide a pleasantly odoured environment as well as masking unpleasant odours. Typically such devices are configured to periodically dispense a burst of liquid fragrance into the atmosphere in the form of an aerosol to act as an air-freshener. It is commonly known to provide aerosol dispensers using flammable gas propellants to dispense the liquid fragrance. However, in recent years there has been a move to mechanical transducers to dispense a liquid spray to address the known problems of flammable gas pellets.

Air-fresheners have been developed to address this issue that generate an aerosol of liquid fragrance using a porous ultrasonic vibration plate. The vibration plate is placed in contact with a wick or capillary tube containing the liquid fragrance, and when actuated the plate vibrates and atomises the fluid from the wick to create and aerosol. The vibrating transducer plates are arranged vertically such that the generated aerosol is directed in a substantially horizontal direction perpendicular to the plane of the plate to project it outwardly into the room. However, it has been found that such arrangements fail to propel the aerosol to a satisfactory distance away from the dispensing unit, and that a concentration of the aerosol spray may remain resident in the atmosphere immediately surrounding the dispenser. This can lead to the deposition of the spray onto the surface of the dispenser.

It has also been found that with vertically orientated transducer plates the replacement of the liquid fragrance container and wick is hindered by the location of the transducer plate in front of the container and the requirement to orientate the wick horizontally. It is also important that the wick is maintained in a permanent and substantive contact with the transducer plate in order for a suitable volume of aerosol to be produced on a consistent basis. It has however been found that there are difficulties experienced by maintenance operatives in achieving this contact when replacing fragrance containers and wicks, and in maintaining this contact generally during the lifecycle of the fragrance cartridge. It is also important that maintenance operatives are able to quickly and easily access the fragrance containers within such dispensers to replace the fragrance containers when they are exhausted.

EP2767774 provides a portable humidifier with an ultrasonic vibrator to which water is fed. A wick is held by a fixed position wick holder. WO2013/159142 provides an atomiser system for a fragrance fed to the piezo disc by a wick. WO2015/050198 is another example of a wick based liquid dispenser.

It is therefore desirable to provide the improved liquid fragrance dispensing apparatus that addresses the above described problems and/or which provides improvements generally.

The invention is directed to a liquid fragrance dispenser comprising:
A) a liquid atomiser including a vibrating atomisation element comprising an actuator and a perforated vibration plate, further comprising an atomiser support including a support plate for supporting the vibration plate in a horizontal orientation;
B) a liquid container provided with;
   a) a wick having a first end located within the liquid container and a second end arranged for engagement with the atomisation element;
   b) a wick support configured to hold and support the wick with the first end located within the liquid container and such that it is movable relative to the liquid container between an extended position and a retracted position with the first end being retained within the liquid container in both positions; and
   c) biasing means arranged to bias the wick to the extended position to urge the wick into engagement with the atomisation element;
   characterised in that:
   the wick support is configured to connect to the atomiser support in an arrangement in which the wick is held in engagement with the vibration plate, and the wick support is configured to be slidingly received beneath the support plate of the atomiser support;
   one of the atomiser support and the wick support comprise at least one guide rail and the other comprises a corresponding at least one guide channel, the at least one guide channel and at least one guide rail configured to guide and hold the wick support beneath the atomiser support plate and arranged such that when the wick is located beneath the vibration plate the distance between the wick support and the atomiser support is such that the wick is compressed towards the retracted position to cause the biasing member to urge the wick against the vibration plate;
   the wick support is connectable to the atomiser support in a first insertion direction and is movable in engagement with the atomiser support to an insertion location at which the wick is aligned with and contacts the vibration plate and the atomiser support includes an engagement surface that slopes downwardly in the direction of the insertion location in the insertion direction arranged to cause compression of the wick towards the retracted position as the wick support is moved towards the insertion position in engagement with the atomiser support.

The biasing member thereby ensures that a constant and substantial contact is maintained between the wick and the vibration element to optimise atomisation.

The atomiser is preferably a piezo electric atomiser.

The biasing means is preferably a spring member operatively connected to the wick either directly or indirectly via an intermediate support member. Preferably the biasing member is arranged at the lower end of the wick in direct engagement with wick. Locating the spring at the base of the wick provides a mechanically simplified arrangement that minimise the number of required parts and simplifies assembly.

The spring member is preferably a cantilever spring having a first end secured to the wick support and a free end secured to the lower end of the wick. The cantilever is preferably a metal spring, which advantageously maintains its sprung properties during prolonged exposure to a liquid fragrance ensuring the there is no loss in biasing force.

The wick support preferably comprises a hollow conical body tapering towards its base and having an opening at the base through which the lower end of the wick extends.

The conical body preferably includes one or more apertures along its length to permit the flow of liquid fragrance inside the wick support to maximise contact between the wick and the liquid.

The wick support preferably includes an upper section located proximate the opening of the liquid container that slidingly receives and guides the wick, and ensures that the base of the wick and the upper end are centrally aligned to ensure the wick is maintained in a vertical orientation.

The biasing element may be engaged with the body section and the support element to bias the support element to the extended position, with the wick being fixed relative to the support element.

The wick may be secured to the support element by one or more projections on the support element engaging with the wick. The one or more projections may be radially provided about the wick and/or on the support member. The one or more projections may have a first state where the wick is absent and a part of the projections extends into the space occupied by the wick in a second state. The one or more projections may be deformed in the second state by the presence of the wick. The separation of the ends of two or more of the projections may be increased in the second state.

The liquid container preferably comprises an opening and the body section is secured to the liquid container such that the support element of the wick support is movably supported within the opening and is movable relative to the liquid container.

The wick is preferably elongate and has a longitudinal axis defined along its length and the wick support is configured to maintain the wick in an orientation in which its longitudinal axis is substantially vertical, the wick being longitudinally movable relative to the liquid container in a vertical direction.

The liquid atomiser is preferably arranged such that the perforated vibration plate is substantially horizontal in use and located such that the perforated vibration plate is in biased contact with the second end of the wick. By orienting the vibration plate horizontally, the liquid container may be located beneath the vibration plate rather than behind it. This allows the liquid container to be accessed by opening the housing and removing the liquid container from beneath the vibration plate with unhindered access. The wick is preferably arranged beneath the vibration plate, and the dispenser further comprises a fan arranged to direct an airflow above the vibration plate on the opposing side to the wick in a direction substantially parallel to the surface of the vibration plate to blow the atomised aerosol away from the location of the vibration plate in a substantially horizontal direction. Combining a fan with the atomiser addresses the issue of limited projection of the atomised fragrance and significantly improves distribution into the space to be serviced by the device.

The fan preferably includes an outlet located proximate the vibration plate to ensure that the airflow is channelled directly over the vibration plate.

The liquid atomiser support and wick support are arranged such that as they are connected the wick is compressed towards the retracted position such that when located beneath the vibration plate is biased towards the extended position into engagement with the vibration plate. In this way wick automatically self-biases on insertion of the liquid fragrance bottle, with no further adjustment being required of the maintenance engineer.

At least one of the wick support and the atomiser support preferably includes a stop formation arranged to locate the wick support relative to the atomiser support in the insertion direction to align the wick and the vibration plate.

The present invention will now be described by way of example only with reference to the following illustrative figures in which:
Figure 1 shows an air fresher according to an embodiment of the invention;
Figure 2 is a cross sectional view of the arrangement of Figure 1;
Figure 3 shows a liquid fragrance bottle and wick support according to another embodiment of the invention;
Figure 4 shows a liquid fragrance bottle and wick support according to a further embodiment of the invention;
Figure 5 is a close up view of the base of the wick support of Figure 4;
Figure 6 shows a view of a liquid fragrance bottle and wick support according to a yet further embodiment of the invention in which a bottle is being inserted into the body of the fragrance;
Figure 7 shows liquid fragrance bottle and wick support of Figure 6 with the bottle fully inserted;
Figure 8a shows an alternative wick support embodiment; and
Figure 8b shows an exploded view of the components of Figure 8a.

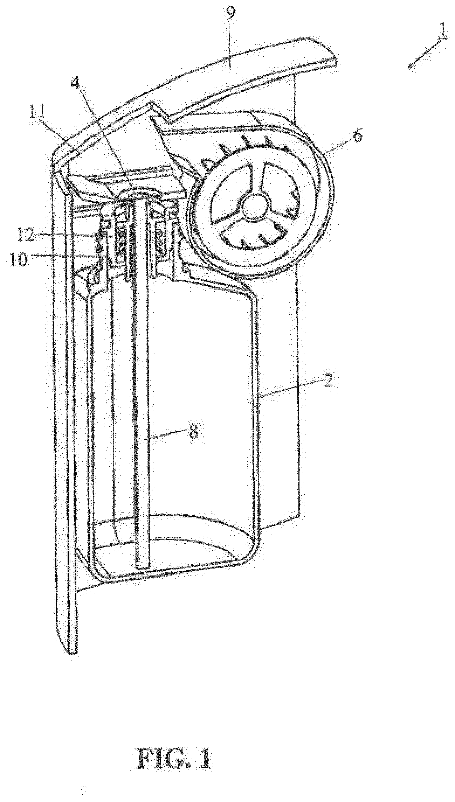

Referring to Figure 1, there is provided a liquid fragrance dispenser 1 comprising a liquid container 2, an actuator 4 and a fan 6 contained within a housing 9. The liquid container 2 is configured to receive and store a volume of liquid fragrance. A wick 8 extends into the container for drawing the liquid fragrance out of the container to a dispensing location. The housing 9 includes a dispensing aperture 11 through which atomised liquid fragrance is dispensed. The container 2 includes a neck 10 defining an opening to the container 2. A wick support 12 is provided within the neck 10 which supports the wick 8 and acts as a bung to close the opening of the neck 10 thereby sealing and closing the container 2. As such, liquid may only leave the container via the wick 8. The wick 8 projects outwardly of the wick support 12 exposing tip arranged for contact with the actuator 4. The wick 8 is preferably substantially ridged and formed of a porous plastic wicking material.

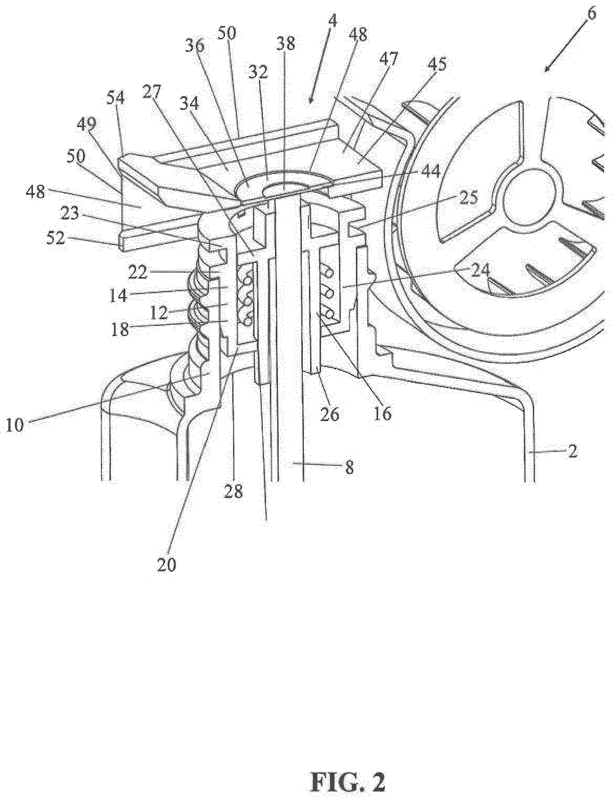

As shown in Figure 2, the wick support 12 includes an outer body section 14 and an inner support section 16. The outer body section 14 includes a lower section 18 configured to fit within the neck 10 of the container 2, having an outer diameter substantially equal to the inner diameter of the neck. The lower section 18 is substantially cylindrical and hollow and includes a tapered leading edge to enable it to be inserted and wedged into the neck 10 of the container 2. The hollow outer body section 14 also includes a base 20. The outer body section 14 is supported on the upper edge of the neck 10 by a shoulder section 22, comprising an outwardly extending annular flange having a diameter greater than the inner diameter of the neck 10. A second annular flange 23 extends outwardly at the upper end of the outer body section 14, axially spaced from the shoulder section 22. A circumferentially extending annular guide channel 25 is defined between the shoulder section 22 and the upper annular flange 23.

A bore 24 is formed within the hollow outer body section 14 that is open at the upper end and which terminates at the lower end at the base 20. The inner support member 16 is received within the bore 24 and comprises a hollow central column 26 within which is received the wick 8. The central column 26 has a diameter less than the inner diameter of the bore 22. The lower end of the column 26 extends through a correspondingly shaped aperture 28 in the base 20 of the outer body section 14 and is able to slide within the aperture 28. The inner support member 14 further includes a substantially disc shaped guide member 27 having a diameter substantially equally to the inner diameter of the bore 24. The flanged guide member 27 extends radially outwards from the column 26 and engages the bore 24 to guide the column 26 as it slides within the bore 22. The support member 16 is therefore able to axially and slidingly translate within the bore 24 of the outer body section 14 carrying with it the wick 8.

The centre column 26 extends axially above the support flange 27. The wick 8 is secured within the centre column 26 and is fixed relative to the column 26, with the tip of the wick 8 extending past the end of the column 26 and past the end of the inner support 16. A compression spring 30 is located within the void defined radially between the centre column 26 and inner wall of the bore 22 of the body section 14 between the support flange 27 and the base 20 of the outer body 14. The spring 30 extends around the centre column 26 and engages the support flange 27 and the base 20 at opposing ends. The spring provides an axially biasing force between the surfaces of the base 20 and the support disc 24 that biases the support 16 to an upwardly extended position away from the base 20.

The actuator 4 comprises a vibration plate 32 in the form of a disc mounted within and supported by an actuator support 34 member. The actuator support 34 holds and supports the vibration plate 32 above the wick 8. The actuator support 34 includes a circular recess for receiving the vibration plate 32 formed in an upper surface 45 of a planar section 47 of the support 34 the vibration plate 32 is preferably a piezoelectric vibration plate configured to vibrate at a high frequency when an electrical current is applied. The vibration plate 32 includes an annular outer section 36 having a first thickness and a thinner central disc section 38 recessed from the annular outer section 36 at the upper surface 45. The annular outer section 36 and the inner disc section 40 are coplanar on the lower surface 42. The vibration plate 32 is seated within the corresponding stepped recess formed in the upper surface 45 of the actuator support 34. Inwardly of the step recess is an aperture 48 is formed through actuator support 34. The aperture 48 is circular and concentric with the vibration plate 32 such that the vibration plate 32 is open to the base of the support 34.

The actuator support 34 comprises a pair of parallel side walls 50 extending downwardly from the planar section 47 defining a channel 48 beneath the upper wall 45. The side walls 50 are arranged parallel to each other and each includes a guide rail 52 extending lengthwise and projecting inwardly into the channel 48 along the lower inner edge of the respective wall 50. The inner edges of the guiderails 52 are laterally spaced by a distance greater than or equal to the inner diameter of the guide channel 25 of the outer body section 14 and less than the outer diameter of the second annular flange 23 and shoulder section 22. The vertical height of the guide rails 52 is configured to be equal to or less than the height of the guide channel 25, defined by the spacing between the second annular flange 23 and shoulder section 22, with guiderails 52 being configured to be received within the guide channels 25. Preferably the guiderails 52 are configured to have a closely toleranced fit within the guide channel 25 such that the outer body member 14 is able to slide along the guiderails 52 but with minimal vertical movement permitted between the guiderails at 52 and the guide channel 25.

As shown in Figure 2, in use the outer body member 14 is inserted into the channel 48 by aligning the guide channel 25 with the guiderails 52. Preferably the distance between the inner surfaces of the side walls 50 is equal to the diameter of the upper second annular flange 23 such that the side walls 50 guide and align the outer body member 14 within the channel 48. The outer body member 14 is slid along the channel 48 to a longitudinal position in which the wick 8 aligns with the centre section 38 of the vibration plate 32. Preferable a stop member, which may be an end wall or stop projection, is provided to longitudinally locate the outer body member 14 at the required position to provide the required alignment between the wick 8 and the vibration plate 32. The distance between the upper surface of the guide rails 52 and the lower surface of the vibration plate 32 is configured to be less than the distance between the lower surface of the upper annular flange 23 and the upper surface of the tip of the wick 8 when the wick 8 is biased to its most upwardly position spring 30. As such, when the wick 8 is aligned with the vibration plate 32 within the channel 48 the height of the tip of the wick 8 is initially greater than the lower surface of the vibration plate 32 with the spring 30 being compressed to reduce the vertical height of the wick 8 to enable it to be accommodated within the vertical height of the channel 48. With the wick 8 compressed, the spring 30 provides a returning force biasing the tip of the wick 8 against the lower surface of the vibration plate 32.

The leading edge of the upper wall 45 proximate the opening 49 to the channel 48 is arranged to define a sloping leading edge such as the mouth of the opening 49 of the channel 48 is flared. The ramped inner surface defined by this sloping leading edge of the upper wall 45 allows for the alignment of the wick support 12 within the channel 48 and also allows the wick support 12 is slide into the channel 48. Engagement of the wick 8 with the sloping inner surface of the ramped front end 54 provides a camming action which wedges the wick 8 downwardly towards the narrowest part of the channel 48 to compress the spring 30 into the compacted biased position. As such, the actuator support 34 and the wick support 12 combine to guide and align the wick 8 with the actuator support plate such that the fragrance bottle may be easily inserted into the air freshener and secured to the actuator support 34 by an operator with the wick 8 compressing on insertion and the vibration plate 32 self-aligning as the bottle 2 is inserted without the requirement for any complex securement or adjustment by the maintenance operative.

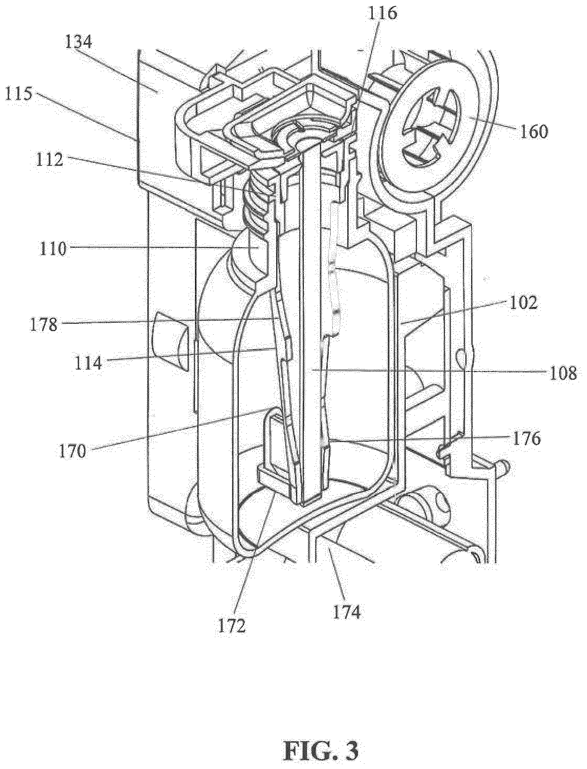

The vibration plate 32 is horizontally orientated, with the wick 8 being arranged vertically in horizontal alignment with vibration plate 32. In contrast to arrangements in the prior art in which the vibration plate is vertically orientated and outwardly facing, the actuator support 34 is arranged within the housing of the air freshener 1 with the vibration plate 32 horizontally oriented and facing upwardly into an air channel within the air freshener 1. As shown in Figure 3, a fan 60 is located within the air freshener housing 9. The fan 60 includes an outlet 62 arranged to direct the air from the fan 60 in a substantially horizontal direction. The fan 60 is arranged proximate the wick support 12 and actuator support 34 with the outlet 62 of the fan 60 being arranged to direct air horizontally above the upper surface 46 of the actuator support 34 in the direction of the front leading edge 54 of the upper wall 45. The upper front edge of the planar member 45 is arranged close to the outlet opening 11 of the air freshener 1.

The vibration plate 32 and fan 60 are connected to a controller which controls actuation of both the fan 60 and the vibration plate 32. The controller actuates the vibration plate 32 on a pre-determined periodic basis to atomise fragrance from the wick 8 in a vertically upwards direction. The fan 60 is controlled such that its operation is synchronised with the vibration plate 32 such that the aerosol of atomised fragrance droplets generated by the vibration plate 32 in a vertically upwards direction are blown horizontally outwardly towards the outlet 11 of the air freshener by the fan 60. Following atomisation the fan 60 is operated for a pre-determined period to purge the resident aerosol from within the air freshener 1 following which the fan operation is terminated until the next atomisation event.

In a further embodiment shown in Figure 3 the wick support 112 includes an outer body member 144 that is received within the neck of the bottle 110. The outer body member 114 is supported on the neck of the bottle 10 by a flange and guide channel arrangement 115 that cooperates with the actuator support 134 as described above. Extending downwardly through the neck 110 and into the bottle 102 is a lower wick support section 170 of the outer body section 114. The lower section 170 is conical in shape tapering towards its base, and extends downwardly substantially to the base of the bottle 102. A spring member 172 is located at the base of the lower section 170 that supports the base of the wick 108. In the arrangement of Figure 3 the spring member 172 is a cantilever member extending horizontally from a lower edge of the lower section 170 and having a free end 174 to which the wick 108 is secured. The spring member 172 is integrally moulded with the outer body member 114. A pair of parallel guide walls 176 vertically guide the lower end of the wick 108. Windows 178 allow fragrance to flow into the lower section 170, and the lower end of the wick 108 extends through the spring member 172 such that the lower tip of the wick 108 is in contact with the fragrance at the base of the bottle 102.

An inner wick support 116 is received within the bore of the outer body member 114. The inner wick support 116 includes an upper surface 117 that closes the neck of the bottle 102. The upper surface 117 includes an aperture 119 that slidingly receives the upper end of the wick 108 to guide the wick 108 as it moves vertically. In a similar manner to the first embodiment described above, the spring member 172 biases the wick 108 to an extended position and in the installed position biases the wick 108 into engagement with the vibration plate.

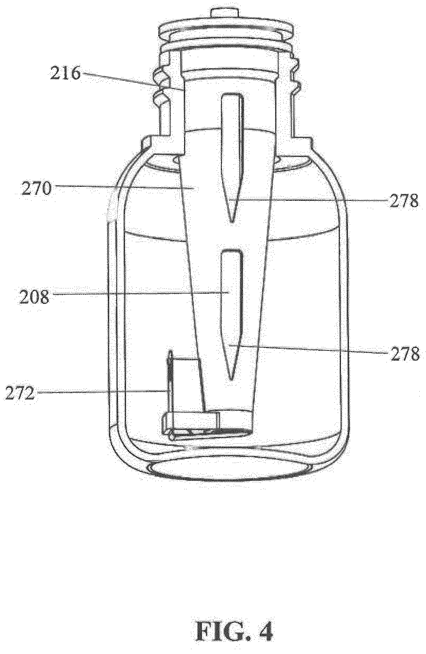

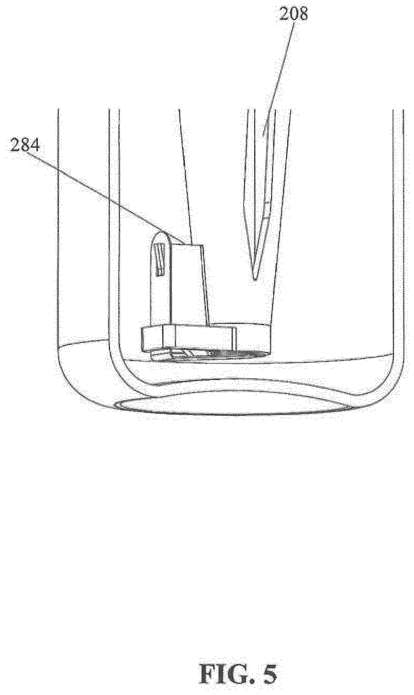

In another embodiment shown in Figure 4, the integrally moulded spring member is replaced by a metal spring member 272. The spring member is formed from a single piece of sprung steel or other sprung metal and is bent along its length through 90 degrees an attachment arm 280 and a spring arm 282, as shown in Figure 5. A support section 284 extends from and is integrally mounted with the base of the lower section 270 of the inner support member 216. The support section 284 includes a retaining projection 286 extending from its upper outer edge. The support arm 182 or the spring member 270 includes an aperture 288 that hooks over the support projection 286 to vertically support the spring member 272. A u-shaped wall 290 laterally restrains the lower end of the support arm 284 to prevent the spring member from pivoting outwardly away from the lower section 270. The spring member 272 is supported such that the spring arm 282 is arranged at a vertically spaced location below an aperture 292 in the base of the conical lower section 270 through which the wick extends, with the lower end of the wick 208 being supported on free and of the spring arm 282. The cantilever spring arm 272 is arranged that downward movement of the free end by the wick 208 generates an upwardly directed biasing force which in biases the wick 208 against the vibration plate.

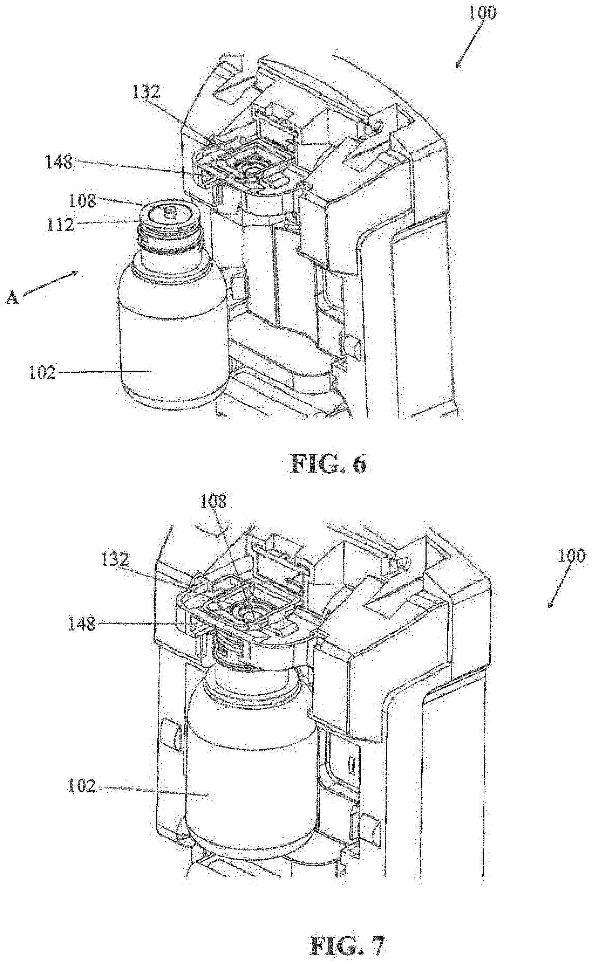

By way of further illustration, Figure 6 shows a bottle 102 being inserted into the body of the fragrance dispenser 100 in an insertion direction A aligned with the longitudinal axis of channel 48. Figure 7 shows the bottle 102 fully inserted with the wick 108 compressed in biased engagement with the vibration plate.

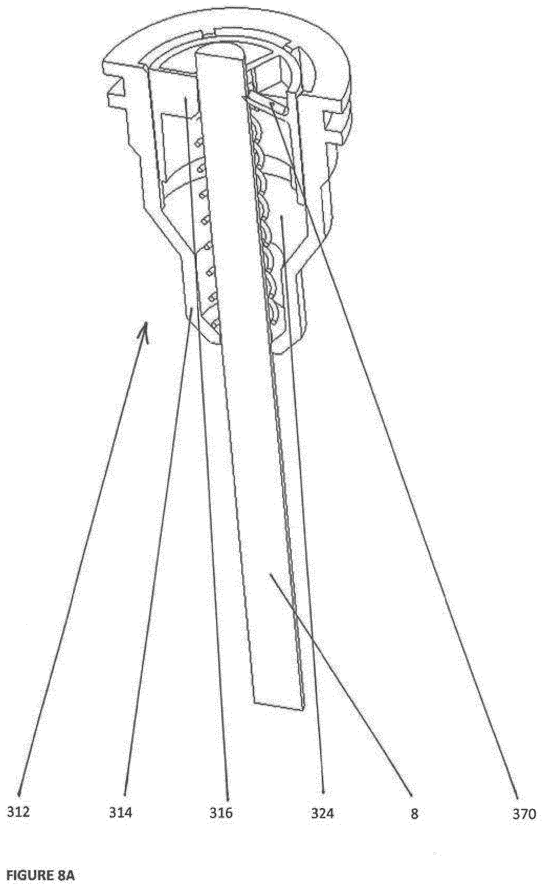

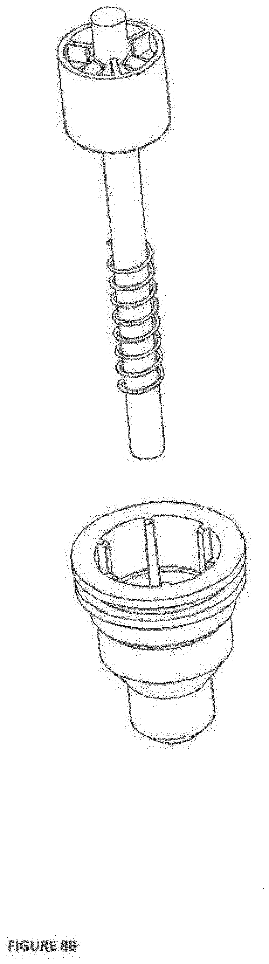

In the Figure 8a and 8b embodiment, the wick support 312 includes an outer body section 314 and an inner support section 316. Once again, the outer body section 314 is designed to fit in the neck 10 of the container 2. The inner support section 316 is similar to the previous embodiments in that the inner support section 316 is able to axially and slideably translate within the bore 324 of the outer body section 314, whilst carrying with it the wick 8.

As with the previous embodiments, the wick 8 is secured within the inner support section 316 so that it is in a fixed position relative to the inner support section 316 and moves with the inner support section 316. In this embodiment, the wick 8 is secured via inwardly extending projections 370. These are inclined relative to the axis of the wick 8 and/or axis of the inner support section 316. The projections 370 engage with and resist movement of the wick 8 within and relative to the inner support section 316, particularly during normal operation. A benefit of this construction and the securing of the wick 8 to the inner support section 316 in the other embodiments is that any increase in dimension of the wick 8 is not transferred to movement of the wick 8 towards the vibration plate 32 or increased pressure on the vibration plate 32. A change in the dimension through the wick 8 is an issue with preferred liquid fragrance as these cause the wick to expand, particularly longitudinally along the long axis of the wick 8. Any change in dimension of the wick is thus limited in its effect to the overwhelming majority part of the wick 8 which is below the inner support section 316 and thus has no negative impact on the vibration plate 32.

## Claims

1. A liquid fragrance dispenser (1) comprising:
A) a liquid atomiser including a vibrating atomisation element comprising an actuator (4) and a perforated vibration plate (32), further comprising an atomiser support including a support plate for supporting the vibration plate (32) in a horizontal orientation;
B) a liquid container (2) provided with;
a) a wick (8) having a first end located within the liquid container (2) and a second end arranged for engagement with the atomisation element;
b) a wick support (12) configured to hold and support the wick (8) with the first end located within the liquid container (2) and such that it is movable relative to the liquid container (2) between an extended position and a retracted position with the first end being retained within the liquid container (2) in both positions; and
c) biasing means arranged to bias the wick (8) to the extended position to urge the wick (8) into engagement with the atomisation element;
**characterised in that**:
the wick support (12) is configured to connect to the atomiser support in an arrangement in which the wick (8) is held in engagement with the vibration plate (32), and the wick support (12) is configured to be slidingly received beneath the support plate of the atomiser support;
one of the atomiser support and the wick support (12) comprise at least one guide rail (52) and the other comprises a corresponding at least one guide channel (25), the at least one guide channel (25) and at least one guide rail (52) configured to guide and hold the wick support (12) beneath the atomiser support plate and arranged such that when the wick (8) is located beneath the vibration plate (32) the distance between the wick support (12) and the atomiser support is such that the wick (8) is compressed towards the retracted position to cause the biasing member to urge the wick (8) against the vibration plate (32);
the wick support (12) is connectable to the atomiser support in a first insertion direction and is movable in engagement with the atomiser support to an insertion location at which the wick (8) is aligned with and contacts the vibration plate (32) and the atomiser support includes an engagement surface that slopes downwardly in the direction of the insertion location in the insertion direction arranged to cause compression of the wick (8) towards the retracted position as the wick support (12) is moved towards the insertion position in engagement with the atomiser support.

2. A liquid fragrance dispenser (1) according to claim 1, wherein the wick support (12) comprises a body section (14) secured to the liquid container (2) and a support element (16) secured to the wick (8), the support element (16) being movably secured to the body section (14) to permit the wick (8) to move between extended and retracted positions, with the wick (8) being fixed relative to the support element (16).

3. A liquid fragrance dispenser (1) according to claim 1 or claim 2, wherein the biasing means is engaged with the body section (14) and the support element (16) to bias the support element (16) to the extended position, with the wick (8) being fixed relative to the support element (16).

4. A liquid fragrance dispenser (1) according to any preceding claim, wherein the wick (8) is arranged beneath the vibration plate (32), and the dispenser (1) further comprises a fan (6) arranged to direct an airflow above the vibration plate (32) on the opposing side to the wick (8) in a direction substantially parallel to the surface of the vibration plate (32) to blow the atomised aerosol away from the location of the vibration plate (32) in a substantially horizontal direction, preferably wherein the fan (6) is arranged proximate the wick support with an outlet of the fan (6) being arranged to direct air horizontally above the upper surface of the vibration plate (32) in the direction of the front leading edge of the upper surface, the front edge of the upper surface being arranged close to the outlet opening of the dispenser (1).

5. A liquid fragrance dispenser (1) according to any preceding claim, wherein the dispenser (1) further comprises a fan (6) to one side of the upper surface of the vibration plate (32) and a dispensing outlet in the housing (9) of the dispenser (1) to the other side of the upper surface of the vibration plate (32), preferably wherein an outlet for the fan (6) is aligned substantially parallel to a surface including the upper surface of the vibration plate (32) and/or the surface including the upper surface of the vibration plate (32) extends to the outlet opening of the dispenser (1) and/or wherein a common axis passes from the outlet for the fan (6) to the outlet opening of the dispenser (1) above the upper surface of the vibration plate (32).

6. A liquid fragrance dispenser (1) according to any preceding claim wherein the wick support (12) includes an upper section located proximate the opening of the liquid container (2) that slidingly receives and guides the wick (8) and/or wherein the biasing means is a spring member arranged at the lower end of the wick (8) in engagement with the with wick (8), preferably wherein the spring member is a cantilever spring having a first end secured to the wick support (12) and a free end secured to the lower end of the wick (8).

7. A liquid fragrance dispenser (1) according to any preceding claim wherein the wick support (12) comprises a hollow conical body tapering towards its base and having an opening at the base through which the lower end of the wick (8) extends, preferably wherein the conical body includes one or more apertures along its length to permit the flow of liquid fragrance inside the wick support (12).

8. A liquid fragrance dispenser (1) according to any preceding claim wherein the liquid container (2) comprises an opening and the wick support (12) is secured to the liquid container (2) such that the wick (8) is vertically movable relative to the liquid container (2), preferably wherein the wick (8) is elongate and has a longitudinal axis defined along its length and the wick support (12) is configured to maintain the wick (8) in an orientation in which its longitudinal axis is substantially vertical, the wick (8) being longitudinally movable relative to the liquid container (2) in a vertical direction, and/or preferably wherein the liquid atomiser is arranged such that the perforated vibration plate (32) is substantially horizontal relative to the vertical orientation of the wick (8) and located such that the perforated vibration plate (32) is in contact with the second end of the wick (8).

9. A liquid fragrance dispenser (1) according to claim 1 wherein at least one of the wick support (12) and the atomiser support includes a stop formation arranged to locate the wick support (12) relative to the atomiser support in the insertion direction to align the wick (8) and the vibration plate (32).

10. A liquid fragrance dispenser (1) according to any preceding claim wherein the vibrating atomisation element is a piezo electric atomiser.

## Patentansprüche

1. Flüssigduftspender (1), umfassend:
A) einen Flüssigkeitszerstäuber, welcher ein vibrierendes Zerstäubungselement aufweist, welches einen Aktuator (4) und eine perforierte Vibrationsplatte (32) umfasst, ferner umfassend einen Zerstäuberhalter, welcher eine Halteplatte zum Halten der Vibrationsplatte (32) in einer horizontalen Ausrichtung aufweist;
B) einen Flüssigkeitsbehälter (2), versehen mit;
a) einem Docht (8) mit einem ersten Ende, welches innerhalb des Flüssigkeitsbehälters (2) angeordnet ist, und einem zweiten Ende, welches zum Eingriff mit dem Zerstäubungselement angeordnet ist;
b) einem Dochthalter (12), welcher ausgestaltet ist, den Docht (8) aufzunehmen und zu halten, wobei das erste Ende innerhalb des Flüssigkeitsbehälters (2) angeordnet ist, und derart, dass er relativ zu dem Flüssigkeitsbehälter (2) zwischen einer ausgefahrenen Position und einer zurückgezogenen Position bewegbar ist, wobei das erste Ende in beiden Positionen innerhalb des Flüssigkeitsbehälters (2) gehalten wird; und
c) Vorspannmittel, welche ausgestaltet sind, den Docht (8) in die ausgefahrene Position vorzuspannen, um den Docht (8) in Eingriff mit dem Zerstäubungselement zu drängen;
**dadurch gekennzeichnet, dass**:
der Dochthalter (12) ausgestaltet ist, mit dem Zerstäuberhalter in einer Anordnung verbunden zu werden, in welcher der Docht (8) in Eingriff mit der Vibrationsplatte (32) gehalten wird, und der Dochthalter (12) ausgestaltet ist, gleitend unter der Trägerplatte des Zerstäuberhalters aufgenommen zu werden;
wobei entweder der Zerstäuberhalter oder der Dochthalter (12) mindestens eine Führungsschiene (52) und der andere einen entsprechenden mindestens einen Führungskanal (25) aufweist, wobei der mindestens eine Führungskanal (25) und die mindestens eine Führungsschiene (52) ausgestaltet sind, den Dochthalter (12) unter der Zerstäuberhalteplatte zu führen und zu halten, und derart angeordnet sind, dass, wenn der Docht (8) unter der Vibrationsplatte (32) angeordnet ist, der Abstand zwischen dem Dochthalter (12) und dem Zerstäuberhalter derart ist, dass der Docht (8) in Richtung der zurückgezogenen Position zusammengedrückt wird, um zu bewirken, dass das Vorspannelement den Docht (8) gegen die Vibrationsplatte (32) drückt;
wobei der Dochthalter (12) mit dem Zerstäuberhalter in einer ersten Einführungsrichtung verbindbar ist und in Eingriff mit dem Zerstäuberhalter zu einer Einführungsstelle bewegbar ist, an welcher der Docht (8) mit der Vibrationsplatte (32) ausgerichtet ist und diese berührt, und wobei der Zerstäuberhalter eine Eingriffsfläche aufweist, welche in der Richtung der Einführungsstelle in der Einführungsrichtung nach unten geneigt ist und angeordnet ist, um ein Zusammendrücken des Dochtes (8) in Richtung der zurückgezogenen Position zu bewirken, wenn der Dochthalter (12) in Richtung der Einführungsstelle in Eingriff mit dem Zerstäuberhalter bewegt wird.

2. Flüssigduftspender (1) nach Anspruch 1, wobei der Dochthalter (12) einen an dem Flüssigkeitsbehälter (2) befestigten Körperabschnitt (14) und ein an dem Docht (8) befestigtes Halteelement (16) umfasst, wobei das Halteelement (16) beweglich an dem Körperabschnitt (14) befestigt ist, um dem Docht (8) zu ermöglichen, sich zwischen ausgefahrener und eingezogener Position zu bewegen, wobei der Docht (8) relativ zu dem Halteelement (16) befestigt ist.

3. Flüssigduftspender (1) nach Anspruch 1 oder Anspruch 2, wobei sich das Vorspannmittel mit dem Körperabschnitt (14) und dem Halteelement (16) in Eingriff befindet, um das Halteelement (16) in die ausgefahrene Position vorzuspannen, wobei der Docht (8) relativ zu dem Halteelement (16) befestigt ist.

4. Flüssigduftspender (1) nach einem der vorhergehenden Ansprüche, wobei der Docht (8) unterhalb der Vibrationsplatte (32) angeordnet ist und der Spender (1) ferner ein Gebläse (6) umfasst, welches angeordnet ist, um einen Luftstrom über die Vibrationsplatte (32) auf der dem Docht (8) gegenüberliegenden Seite in eine Richtung im Wesentlichen parallel zu der Oberfläche der Vibrationsplatte (32) zu leiten, um das zerstäubte Aerosol von der Stelle der Vibrationsplatte (32) in einer im Wesentlichen horizontalen Richtung wegzublasen, wobei das Gebläse (6) vorzugsweise in der Nähe des Dochthalters angeordnet ist, wobei ein Auslass des Gebläses (6) angeordnet ist, um Luft horizontal über die obere Fläche der Vibrationsplatte (32) in die Richtung der vorderen Vorderkante der oberen Fläche zu leiten, wobei die vordere Kante der oberen Fläche nahe der Auslassöffnung des Spenders (1) angeordnet ist.

5. Flüssigduftspender (1) nach einem der vorhergehenden Ansprüche, wobei der Spender (1) ferner ein Gebläse (6) auf einer Seite der oberen Fläche der Vibrationsplatte (32) und einen Spenderauslass in dem Gehäuse (9) des Spenders (1) auf der anderen Seite der oberen Fläche der Vibrationsplatte (32) umfasst, wobei vorzugsweise ein Auslass für das Gebläse (6) im Wesentlichen parallel zu einer Fläche ausgerichtet ist, welche die obere Fläche der Vibrationsplatte (32) aufweist, und/oder die Fläche, welche die obere Fläche der Vibrationsplatte (32) aufweist, sich zu der Auslassöffnung des Spenders (1) erstreckt, und/oder wobei eine gemeinsame Achse von dem Auslass für das Gebläse (6) zu der Auslassöffnung des Spenders (1) oberhalb der oberen Fläche der Vibrationsplatte (32) verläuft.

6. Flüssigduftspender (1) nach einem der vorhergehenden Ansprüche, wobei der Dochthalter (12) einen in der Nähe der Öffnung des Flüssigkeitsbehälters (2) angeordneten oberen Abschnitt aufweist, welcher den Docht (8) gleitend aufnimmt und führt, und/oder wobei das Vorspannmittel ein Federelement ist, welches an dem unteren Ende des Dochtes (8) in Eingriff mit dem Docht (8) angeordnet ist, wobei das Federelement vorzugsweise eine freitragende Feder ist, welche ein erstes Ende aufweist, welches an dem Dochthalter (12) befestigt ist, und ein freies Ende, welches an dem unteren Ende des Dochtes (8) befestigt ist.

7. Flüssigduftspender (1) nach einem der vorhergehenden Ansprüche, wobei der Dochthalter (12) einen hohlen konischen Körper umfasst, welcher sich zu seiner Basis hin verjüngt und an der Basis eine Öffnung aufweist, durch welche sich das untere Ende des Dochtes (8) erstreckt, wobei der konische Körper vorzugsweise eine oder mehrere Öffnungen entlang seiner Länge aufweist, um den Fluss von Flüssigduft innerhalb des Dochthalters (12) zu ermöglichen.

8. Flüssigduftspender (1) nach einem der vorhergehenden Ansprüche, wobei der Flüssigkeitsbehälter (2) eine Öffnung umfasst und der Dochthalter (12) an dem Flüssigkeitsbehälter (2) derart befestigt ist, dass der Docht (8) relativ zu dem Flüssigkeitsbehälter (2) vertikal beweglich ist, wobei der Docht (8) vorzugsweise länglich ist und eine Längsachse aufweist, welche entlang seiner Länge definiert ist, und der Dochthalter (12) ausgestaltet ist, den Docht (8) in einer Ausrichtung zu halten, in welcher seine Längsachse im Wesentlichen vertikal ist, wobei der Docht (8) in Längsrichtung relativ zu dem Flüssigkeitsbehälter (2) in einer vertikalen Richtung beweglich ist, und/oder wobei vorzugsweise der Flüssigkeitszerstäuber derart angeordnet ist, dass die perforierte Vibrationsplatte (32) im Wesentlichen horizontal relativ zu der vertikalen Ausrichtung des Dochtes (8) ist und derart angeordnet ist, dass die perforierte Vibrationsplatte (32) in Kontakt mit dem zweiten Ende des Dochtes (8) ist.

9. Flüssigduftspender (1) nach Anspruch 1, wobei mindestens einer von dem Dochthalter (12) und dem Zerstäuberhalter eine Anschlaganordnung aufweist, welche ausgestaltet ist, den Dochthalter (12) relativ zu dem Zerstäuberhalter in der Einführrichtung zu positionieren, um den Docht (8) und die Vibrationsplatte (32) auszurichten.

10. Flüssigduftspender (1) nach einem der vorhergehenden Ansprüche, wobei das vibrierende Zerstäubungselement ein piezoelektrischer Zerstäuber ist.

## Revendications

1. Distributeur de parfum liquide (1) comprenant :
A) un atomiseur de liquide comportant un élément d'atomisation vibrant comprenant un actionneur (4) et une plaque de vibration perforée (32), comprenant en outre un support d'atomiseur comportant une plaque de support pour supporter la plaque de vibration (32) dans une orientation horizontale ;
B) un récipient de liquide (2) pourvu :
a) d'une mèche (8) ayant une première extrémité située à l'intérieur du récipient de liquide (2) et une deuxième extrémité agencée pour s'engager avec l'élément d'atomisation ;
b) d'un support de mèche (12) configuré pour retenir et supporter la mèche (8) avec la première extrémité située à l'intérieur du récipient de liquide (2) et de sorte qu'il soit mobile par rapport au récipient de liquide (2) entre une position étendue et une position rétractée avec la première extrémité retenue à l'intérieur du récipient de liquide (2) dans les deux positions ; et
c) d'un moyen de sollicitation agencé pour solliciter la mèche (8) vers la position étendue pour pousser la mèche (8) en engagement avec l'élément d'atomisation ;
**caractérisé en ce que** :
le support de mèche (12) est configuré pour se relier au support d'atomiseur dans un agencement dans lequel la mèche (8) est retenue en engagement avec la plaque de vibration (32), et le support de mèche (12) est configuré pour être reçu de manière coulissante en dessous de le plaque de support du support d'atomiseur ;
l'un parmi le support d'atomiseur et le support de mèche (12) comprend au moins un rail de guidage (52) et l'autre comprend au moins un canal de guidage (25) correspondant, l'au moins un canal de guidage (25) et l'au moins un rail de guidage (52) étant configurés pour guider et retenir le support de mèche (12) en dessous de la plaque de support d'atomiseur et agencés de sorte que, lorsque la mèche (8) est située en dessous de la plaque de vibration (32), la distance entre le support de mèche (12) et le support d'atomiseur soit telle que la mèche (8) est comprimée vers la position rétractée pour amener l'élément de sollicitation à pousser la mèche (8) contre la plaque de vibration (32) ;
le support de mèche (12) peut être relié au support d'atomiseur dans une première direction d'insertion et est mobile en engagement avec le support d'atomiseur jusqu'à un emplacement d'insertion au niveau duquel la mèche (8) est alignée et en contact avec la plaque de vibration (32) et le support d'atomiseur comporte une surface d'engagement qui s'incline vers le bas dans la direction de l'emplacement d'insertion dans la direction d'insertion agencée pour provoquer la compression de la mèche (8) vers la position rétractée à mesure que le support de mèche (12) est déplacé vers la position d'insertion en engagement avec le support d'atomiseur.

2. Distributeur de parfum liquide (1) selon la revendication 1, dans lequel le support de mèche (12) comprend une section de corps (14) fixée au récipient de liquide (2) et un élément de support (16) fixé à la mèche (8), l'élément de support (16) étant fixé de manière mobile à la section de corps (14) pour permettre à la mèche (8) de se déplacer entre des positions étendue et rétractée, avec la mèche (8) fixe par rapport à l'élément de support (16).

3. Distributeur de parfum liquide (1) selon la revendication 1 ou 2, dans lequel le moyen de sollicitation est engagé avec la section de corps (14) et l'élément de support (16) pour solliciter l'élément de support (16) vers la position étendue, avec la mèche (8) fixe par rapport à l'élément de support (16).

4. Distributeur de parfum liquide (1) selon l'une des revendications précédentes, dans lequel la mèche (8) est agencée en dessous de la plaque de vibration (32), et le distributeur (1) comprend en outre un ventilateur (6) agencé pour diriger un flux d'air au-dessus de la plaque de vibration (32) sur le côté opposé à la mèche (8) dans une direction essentiellement parallèle à la surface de la plaque de vibration (32) pour souffler l'aérosol atomisé à distance de l'emplacement de la plaque de vibration (32) dans une direction essentiellement horizontale, où de préférence le ventilateur (6) est agencé à proximité du support de mèche avec une sortie du ventilateur (6) agencée pour diriger l'air horizontalement au-dessus de la surface supérieure de la plaque de vibration (32) dans la direction du bord d'attaque avant de la surface supérieure, le bord avant de la surface supérieure étant agencé près de l'ouverture de sortie du distributeur (1).

5. Distributeur de parfum liquide (1) selon l'une des revendications précédentes, dans lequel le distributeur (1) comprend en outre un ventilateur (6) sur un côté de la surface supérieure de la plaque de vibration (32) et une sortie de distribution dans le boîtier (9) du distributeur (1) sur l'autre côté de la surface supérieure de la plaque de vibration (32), où de préférence une sortie pour le ventilateur (6) est alignée de manière essentiellement parallèle à une surface comportant la surface supérieure de la plaque de vibration (32) et/ou la surface comportant la surface supérieure de la plaque de vibration (32) s'étend jusqu'à l'ouverture de sortie du distributeur (1) et/ou où un axe commun passe de la sortie pour le ventilateur (6) à l'ouverture de sortie du distributeur (1) au-dessus de la surface supérieure de la plaque de vibration (32).

6. Distributeur de parfum liquide (1) selon l'une des revendications précédentes dans lequel le support de mèche (12) comporte une section supérieure située à proximité de l'ouverture du récipient de liquide (2) qui reçoit et guide de manière coulissante la mèche (8) et/ou où le moyen de sollicitation est un élément de ressort agencé au niveau de l'extrémité inférieure de la mèche (8) en engagement avec la mèche (8), où de préférence l'élément de ressort est un ressort en porte-à-faux ayant une première extrémité fixée au support de mèche (12) et une extrémité libre fixée à l'extrémité inférieure de la mèche (8).

7. Distributeur de parfum liquide (1) selon l'une des revendications précédentes, dans lequel le support de mèche (12) comprend un corps conique creux se rétrécissant vers sa base et ayant une ouverture au niveau de la base à travers laquelle s'étend l'extrémité inférieure de la mèche (8), où de préférence le corps conique comporte un ou plusieurs orifice(s) sur sa longueur pour permettre l'écoulement de parfum liquide à l'intérieur du support de mèche (12).

8. Distributeur de parfum liquide (1) selon l'une des revendications précédentes, dans lequel le récipient de liquide (2) comprend une ouverture et le support de mèche (12) est fixé au récipient de liquide (2) de sorte que la mèche (8) soit mobile verticalement par rapport au récipient de liquide (2), où de préférence la mèche (8) est allongée et a un axe longitudinal défini sur sa longueur et le support de mèche (12) est configuré pour maintenir la mèche (8) dans une orientation dans laquelle son axe longitudinal est essentiellement vertical, la mèche (8) étant longitudinalement mobile par rapport au récipient de liquide (2) dans une direction verticale, et/ou où de préférence l'atomiseur de liquide est agencé de sorte que la plaque de vibration perforée (32) soit essentiellement horizontale par rapport à l'orientation verticale de la mèche (8) et situé de sorte que la plaque de vibration perforée (32) soit en contact avec la deuxième extrémité de la mèche (8).

9. Distributeur de parfum liquide (1) selon la revendication 1 dans lequel au moins l'un parmi le support de mèche (12) et le support d'atomiseur comporte une formation d'arrêt agencée pour positionner le support de mèche (12) par rapport au support d'atomiseur dans la direction d'insertion pour aligner la mèche (8) et la plaque de vibration (32).

10. Distributeur de parfum liquide (1) selon l'une des revendications précédentes dans lequel l'élément d'atomisation vibrant est un atomiseur piézoélectrique.
